# EUROPÄISCHE PATENTSCHRIFT

(11) **EP 3 142 969 B1**
(45) Veröffentlichungstag und Bekanntmachung des Hinweises auf die Patenterteilung: **03.07.2019**
(21) Anmeldenummer: 15720720.0
(22) Anmeldetag: 05.05.2015
(51) Int. Cl.: C02F 1/72, A01N 59/02, A61L 2/18, B08B 9/032, C02F 1/50, C02F 1/66

(54) **VERFAHREN ZUR HYGIENISIERUNG VON MOBILEN TRINKWASSERANLAGEN**
METHOD FOR SANITIZING MOBILE POTABLE WATER UNITS
PROCÉDÉ D'HYGIÉNISATION D'INSTALLATIONS MOBILES D'EAU POTABLE

(30) Priorität: 16.05.2014 DE 102014209336
(43) Veröffentlichungstag der Anmeldung: 22.03.2017
(73) Patentinhaber: Carela GmbH, 79618 Rheinfelden (DE)
(72) Erfinder: KRUMREY, Bernd, 79591 Eimeldingen (DE); REISGYS, Michael, CH-4132 Muttenz (CH)
(74) Vertreter: Friese Goeden Patentanwälte PartGmbB
(86) Internationale Anmeldenummer: PCT/EP2015/059854
(87) Internationale Veröffentlichungsnummer: WO 2015/173076

(56) Entgegenhaltungen:
- WO-A1-93/02973
- WO-A1-2009/071664
- DE-A1-102007 003 748
- GOSSELIN F ET AL: "Drinking water and biofilm disinfection by Fenton-like reaction", WATER RESEARCH, Bd. 47, Nr. 15, 29. Juni 2013 (2013-06-29) , Seiten 5631-5638, XP028726168, ISSN: 0043-1354, DOI: 10.1016/J.WATRES.2013.06.036

## Beschreibung

Die Erfindung betrifft die Verbesserung der Bereitstellung von Trinkwasser hoher Qualität an Orten, an denen eine Versorgung von Personen und auch Tieren mit Wasser der erforderlichen Qualität aus einem intakten Rohrnetz gar nicht oder wenigstens zeitweise nicht möglich ist, weshalb zur Gewährleistung der Wasserbereitstellung das Wasser mit Hilfe mobiler Anlagen von ferngelegenen Orten heran transportiert werden muss und/oder durch Aufbereitung von Wasser aus lokalen Wasserquellen, die nur Wasser liefern, das unbehandelt bzw. unaufbereitet nicht genießbar ist, z.B. Rohwasser, unsauberes Brunnenwasser und Oberflächenwasser wie Brackwasser, Flusswasser oder Meerwasser, erzeugt werden muss. Genauer betrifft die Erfindung ein Verfahren zur Hygienisierung von mobilen Trinkwasseranlagen.

Beispiele für Situationen, bei denen die Versorgung mit Trinkwasser unter Einsatz mobiler Anlagen gewährleistet werden muss, sind Hilfseinsätze in Krisengebieten, z.B. in Gebieten, in denen kriegerische Auseinandersetzungen herrschen oder die Folgen von Naturkatastrophen bewältigt werden müssen, so dass die Funktionsfähigkeit der Infrastruktur gestört ist, aber auch die Versorgung von z.B. Forschungsstationen in Gebieten ohne jegliche lokale Infrastruktur oder die Versorgung von Menschenansammlungen bei Großveranstaltungen, in Flüchtlingslagern oder auch bei Militärmanövern außerhalb von Ortschaften.

Wenn im Rahmen der vorliegenden Beschreibung von "Trinkwasser" gesprochen wird, soll damit nicht nur Wasser gemeint sein, das tatsächlich nur zum Trinken, d.h. als Lebensmittel, dient, sondern als "Trinkwasser" soll auch ein Reinwasser für andere Verwendungen, die ein reines, aufbereitetes Wasser erfordern, ohne dass dieses notwendiger Weise die gleichen strengen Anforderungen erfüllen muss, wie sie an das Lebensmittel Wasser gestellt werden, bezeichnet werden.

Am Beispiel der Gewährleistung der Versorgung von Personen mit Trinkwasser in Krisengebieten durch Hilfsorganisationen werden nachfolgend einige der Probleme, die beim Einsatz mobiler Anlagen zur Trinkwasserversorgung entstehen können und die die Versorgung mit qualitativ einwandfreiem Trinkwasser gefährden können, etwas ausführlicher geschildert.

Eine wichtige Aufgabe von Hilfsorganisationen ist es, in Krisengebieten hygienisch einwandfreies Trinkwasser bereitzustellen. Dazu werden mobile Trinkwasseraufbereitungs- und Speicheranlagen eingesetzt, welche zweckmäßiger weise auf Lastkraftwagen, -anhängern, in Überseecontainern oder auf Schiffen montiert sind.

Viele der Krisengebiete der Welt liegen in Klimazonen mit durchgängig oder zeitweise hohen Außentemperaturen. Da sich pathogene Keime wie z.B. die beispielhaft zu nennenden Problemkeime Legionella pneumophila oder Pseudomonas aeruginosa bevorzugt in der Wärme entwickeln, wird unter solchen Bedingungen die Beschaffung und Bevorratung von Trinkwasser einer hohen hygienischen Qualität zu einer besonderen Herausforderung.

Ein wichtiger Problemfaktor sind dabei Biofilme, die sich an den wasserberührten Flächen der Anlage ausbilden und eine erhebliche Widerstandskraft aufweisen können. Biofilme sind dünne Filme mikrobiellen Ursprungs, die häufig nicht oder nicht sofort optisch erkennbar sind. An den trinkwasserberührten Oberflächen wachsen sie bei erhöhten Temperaturen besonders schnell, und wenn sie einmal entstanden sind, bieten sie unter dichten widerstandsfähigen Schleimschichten Schutz und Rückzugsmöglichkeiten für zahlreiche pathogene Keime, so dass diese von den meisten bekannten Trinkwasserdesinfektionsmitteln wie z. B. Chlor und Chlordioxid nur ungenügend erreicht werden. Folglich kommt der Entfernung des Biofilms an den trinkwasserberührten Oberflächen für die hygienische Qualität, insbesondere eine möglichst langdauernde hygienische Qualität, des Trinkwassers eine besondere Bedeutung zu.

Das Dokument DE 10 2007 0003 748 A1 offenbart ein bekanntes Verfahren zur Hygienisierung von Trinkwasseranlagen.

Gemäß dem Stand der Technik werden die mobilen Trinkwasseranlagen und -behälter zur Reinigung und Desinfektion üblicherweise mit einer wässrigen Natriumhypochlorit-Lösung ("Chlorlauge") behandelt. Dazu werden Rohrleitungen, Pumpen und weitere Anlagenteile mit einer solchen Lösung gefüllt, die man die erforderliche Zeit einwirken lässt. Auf die Oberflächen der geleerten Trinkwasserbehälter wird die Natriumhypochlorit-Lösung mit manuellen Sprühgeräten aufgetragen, und danach wird mit Bürsten, Schwämmen und ähnlichen Arbeitsgeräten mechanisch nachgearbeitet.

Hierbei müssen die mit der Reinigung und Desinfektion befassten Personen einerseits aufgrund der ätzenden und bleichenden Wirkung des Natriumhypochlorits und andererseits aufgrund der Freisetzung von Chlorgas aufwändige Haut- und Atemschutzausrüstungen tragen. Die Personen sind zusätzlich durch das warme Klima, die Enge in den knapp bemessenen Behältern sowie den beengenden Schutzausrüstungen besonders schweren Belastungen ausgesetzt. Es wäre daher wünschenswert, wenn man die Reinigung und Desinfektion arbeitstechnisch einfacher gestalten könnte, ohne im Hinblick auf den Reinigungs- und Desinfektionserfolg Abstriche machen zu müssen.

Die derzeit erforderlichen aggressiven Reinigungs- und Desinfektionstechniken unter Einsatz chlorhaltiger Mittel, insbesondere unter Einsatz von wässrigen Natriumhypochlorit-lösungen, führen außerdem auch zu einer unerwünschten Beanspruchung des Materials der Anlagen und ihrer technischen Teile. Mobile Trinkwasseranlagen und -behälter sind häufig in Edelstahl ausgeführt. Eine Natriumhypochlorit-Lösung wirkt aufgrund ihres hohen Chloridgehaltes und ihrer Oxidationskraft besonders korrosiv auf den Werkstoff Edelstahl. Die Anlagen haben daher bei der oftmals häufig zu wiederholenden Anwendung von Lösungen von Natriumhypochlorit nur eine begrenzte Standzeit, ebenso eine verkürzte Lebensdauer. Kostspielige Reparaturen oder entsprechend hohe Investitionen für eine Neubeschaffung sind die Folge.

Ferner ist Natriumhypochlorit ein Gefahrgut("Ätzend"), weshalb beim Transport und der Lagerung hohe Sicherheitsauflagen zu beachten sind.

Natriumhypochlorit ist auch umweltgefährlich. Die Entsorgung der Spülwässer nach einer Reinigung und Desinfektion ist daher problematisch, was insbesondere für Krisengebiete ohne funktionierende Infrastruktur gilt.

Die handelsübliche Natriumhypochlorit-Lösung ist nur eingeschränkt lagerstabil. Der Gehalt an freiem Chlor nimmt mit der Zeit ab. Dieser Zerfallsprozess wird durch erhöhte Temperaturen, wie sie in klimatisch warmen Regionen vorherrschen, beschleunigt.

Da in vielen Fällen der Alterungsgrad und damit die aktuelle Aktivität der zu verwendenden Lösung ohne genauere Analysen nicht einfach feststellbar ist, und unter praktischen Einsatzbedingungen meist auf besondere Analysen verzichtet wird, werden die Natriumhypochlorit-Behandlungslösungen in der Praxis häufig überdosiert, was nicht nur unnötige Kosten verursacht, sondern auch Anlagen und Umwelt unnötig belastet.

Es ist daher Aufgabe der vorliegenden Erfindung, ein neuartiges Verfahren zur Reinigung und Hygienisierung von mobilen Trinkwasseranlagen und -behältern zu schaffen, welches die Nachteile der bisherigen Verfahren unter Einsatz von wässrigen Natriumhypochlorit-Lösungen nicht aufweist.

Insbesondere soll das Verfahren mit maßgeschneiderten Mitteln durchführbar sein, die für den jeweiligen Einsatzzweck einfach korrekt dosierbar sind, in der Anwendung, beim Transport und bei der Lagerung stabiler und weniger gefährlich sind als wässrige Natriumhypochlorit-Lösungen und die das Reinigungspersonal vor geringere Anforderungen stellen und dieses und die Umwelt weniger belasten.

Diese Aufgabe wird durch ein Verfahren gemäß den Ansprüchen 1 und 2 gelöst.

Der Begriff "Hygienisierung", der in der vorliegenden Anmeldung zur Bezeichnung des angestrebten Behandlungserfolgs verwendet wird, bedeutet im Rahmen dieser Beschreibung die Erzeugung von hygienischen, einwandfrei sauberen Oberflächen unter Entfernung von biologischen Ablagerungen/Biofilmen und das Inaktivieren von darin enthaltenen pathogenen Keimen.

Der Begriff beinhaltet daher mehr als der Begriff "Desinfizieren", der sich normalerweise nicht auf die Entfernung von Biofilmen und anderen mikrobiellen und/oder mineralischen Ablagerungen bezieht.

Die Aufgabenstellung wird durch ein Verfahren gelöst, welchem ein Set (Kit) von mehreren chemisch und mengenmäßig aufeinander abgestimmten chemischen Produkten zu Grunde liegt, insbesondere von mindestens zwei Komponenten, aus denen durch Mischen vor Ort oder Einmischen in Wasser vor Ort die eigentliche Behandlungslösung hergestellt wird, sowie mindestens einer weiteren, ebenfalls chemisch und mengenmäßig auf die Mischung der beiden ersten Komponenten abgestimmten Lösung, die dazu dient, die verbrauchte Behandlungslösung bzw. Spüllösung nach ihrem Einsatz sicher und vollständig zu neutralisieren, worunter hierin eine Überführung der aktiven Bestandteile in unschädliche, weitgehend pH-neutrale und redox-neutrale Reaktionsprodukte, die keine Umweltgefährdung darstellen, verstanden werden soll.

Das Set ist auf die Baugröße der jeweiligen zu hygienisierenden mobilen Trinkwasser-Anlagen und -Behälter abgestimmt. In der Praxis ist aufgrund von Normung bzw. technischen Vorschriften für mobile Trinkwasseranlagen und von technischen Traditionen nur eine geringe Anzahl von Baugrößen anzutreffen, so dass die Anpassung an die jeweiligen Baugrößen einfach durch Bereitstellung einer geringen Anzahl verschiedener Sets erfolgen kann. So ist die Anwendung für das ausführende Personal einfach, sicher und schnell.

Das Set beinhaltet drei Komponenten: zwei Komponenten ergeben zusammen mit Wasser die einsatzbereite Hygienisierungslösung. Die dritte Komponente dient der Neutralisation und Deaktivierung der Spülwässer nach dem Hygienisierungsprozess.

Alle drei Komponenten sind unvermischt nahezu unbegrenzt haltbar, so dass kein Grund für Unsicherheiten bezüglich der Aktivität der daraus hergestellten Behandlungslösungen besteht. Eine vorsorgliche Überdosierung kann dadurch vermieden werden.

Die erste Komponente ist eine saure, vorzugsweise mineralsaure feste Komponente oder eine flüssige Komponente in Form eines wässrigen Reinigungskonzentrats mit einem pH-Wert < 3.

Als saure Wirkstoffe werden Mineralsäuren, und zwar Phosphorsäure, Schwefelsäure, Salpetersäure, besonders bevorzugt Schwefelsäure und Phosphorsäure, eingesetzt, da diese von Mikroorganismen nicht verwertbar sind und daher nicht als potentielle Nährsubstrate wirken. Von den Mineralsäuren gehört Salzsäure aufgrund ihrer korrosiven Wirkung auf Edelstähle nicht zu den eingesetzten Mineralsäuren.

Die Säurekomponente kann auch Säurelieferanten oder -bildner in Form eines oder mehrerer saurer Salze enthalten. Als saure Salze können bevorzugt Mineralsalze von mehrbasigen Mineralsäuren eingesetzt werden, z.B. Alkalimetallhydrogenphosphate und/oder -hydrogensulfate. Bevorzugt sind Alkalimetallhydrogensulfate, und besonders bevorzugt ist Natriumhydrogensulfat.

Die saure Komponente kann, wie grundsätzlich auch die anderen Komponenten, anwendungstechnische Hilfsstoffe enthalten wie z.B. Tenside, Komplexbildner, Alkohole, Polyglycole.

Die zweite Komponente enthält ein starkes halogenfreies Oxidationsmittel, dem für die Zerstörung und Auflösung der im Rahmen der Hygienisierung zu beseitigenden Biofilme entscheidende Bedeutung zukommt, und zwar Alkalimetallpercarbonate, -peroxide und -perborate, wobei jedoch bevorzugt Wasserstoffperoxid direkt in wässriger Lösung eingesetzt wird. Wasserstoffperoxid ist bei seiner Verwendung als zweite Komponenten mit bekannten Stabilisatoren auszurüsten, um, beispielsweise durch Maskierung schädlicher Metallionen, Zerfallsprozesse zu inhibieren, so dass eine ausreichende Lagerstabilität auch unter warmen klimatischen Bedingungen im Set gegeben ist. Die dritte Komponente enthält alkalische Stoffe und Reduktionsmittel in Kombination. Alkalische Stoffe sind Alkalimetallhydroxide, -carbonate, -hydrogencarbonate und/oder basische Alkalimetallphosphate. Besonders bevorzugt sind Alkalimetallcarbonate und/oder -hydrogen-carbonate, und besonders bevorzugt ist Natriumhydrogen-carbonat.

Als Reduktionsmittel haben sich in der dritten Komponente reduzierende anorganische Schwefelverbindungen bewährt, die z.B. den Vorteil aufweisen, dass die aus ihnen durch Oxidation entstehenden Reaktionsprodukte, insbesondere Sulfate, nicht toxisch sind und umweltverträglich sind, nämlich Alkali- und Erdalkalisalze der dithionigen Säure, der schwefligen Säure, der dischwefligen Säure und der Thioschwefelsäure. Die reduzierenden Salze können, wenn sie in Lösung miteinander verträglich sind, zur Bereitstellung des gewünschten Reduktionsvermögens auch in beliebigen Mischungen zur Anwendung kommen. Besonders bewährt haben sich Salze der schwefligen und der dischwefligen Säure, insbesondere ihre Alkalimetalsalze wie z. B. Natriumsulfit, Natriumhydrogensulfit und Natriumdisulfit.

In einem erfindungsgemäßen Set sind die drei genannten Komponenten in solchen aufeinander abgestimmten Mengen enthalten, dass für ihre Verwendung zur Hygienisierung keine zusätzlichen Abmess- und Dosierungsschritte durchgeführt werden müssen.

Da vorgesehen ist, dass die Absolutmengen der drei Komponenten, in Anpassung des Sets an die Größe der zu hygienisierenden mobilen Trinkwasseranlage, variieren können, und die einzelnen Komponenten im Set in fester Form oder auch in Form von mehr oder weniger stark mit Wasser verdünnten flüssigen Konzentraten vorliegen können, werden die Relativmengen der Komponenten des Sets am besten indirekt dadurch beschrieben, dass man die Mengen anhand der Eigenschaften einer aus ihnen erzeugten Hygienisierungslösung, bezogen auf eine Standard-Wassermenge, insbesondere eine Menge von 100 l Wasser, definiert.

Die Mengen der ersten und der zweiten Komponente in einem Set sind so gewählt, dass durch vollständiges Auflösen oder Einmischen der ersten und zweiten Komponente des Sets in Wasser 100 l einer wässrigen Hygienisierungslösung gebildet werden, deren pH einen Wert von 3 oder weniger, vorzugsweise zwischen 1,5 und 2,5, aufweist, und die ein Oxidationsvermögen bzw. einen Gehalt an aktivem Sauerstoff aufweist, die einem Wasserstoffperoxidgehalt von 650 bis 900 ppm Wasserstoffperoxid in 100 l der wässrigen Hygienisierungslösung entsprechen.

Die dritte Komponente ist so bemessen, dass der alkalische Bestandteil stöchiometrisch zur vollständigen Neutralisierung der sauren Bestandteile der ersten Komponente bzw. der fertig gemischten Hygienisierungslösung ausreicht, während die Menge des Reduktionsmittels so gewählt ist, dass die gesamte Menge an aktivem Sauerstoff, ausgedrückt als Wasserstoffperoxidgehalt, bezogen auf 100 l fertiger Hygienisierungslösung, unter Bildung von Wasser reduziert wird.

Da bei der Hygienisierung bereits mehr oder weniger große Anteile der sauren Bestandteile oder des Aktivsauerstoffs neutralisiert bzw. verbraucht werden können, sollte die Bereitstellung stöchiometrischer Neutralisierungsmengen in der dritten Komponente eines Sets auf jeden Fall ausreichen. Gegebenenfalls können jedoch, auf der Basis empirisch ermittelter Werte, auch leicht unterstöchiometrische Mengen vorgesehen werden, oder aber, z.B. zur Beschleunigung und schnelleren Vervollständigung der Neutralisierungsreaktionen, auch etwas überstöchiometrische Mengen.

Sind die in einem Set bereit gestellten Mengen der Komponenten für die Herstellung von mehr, oder auch von weniger, als 100 l wässriger Hygienisierungslösung bestimmt, werden die auf 100 l bezogenen Mengen einfach proportional vergrößert oder verkleinert.

Sind die erste und die zweite Komponente des Sets Feststoffe, wird die gewünschte Hygienisierungslösung durch einfaches Auflösen der ersten und zweiten Komponente in der in einer begleitenden Gebrauchsanweisung angegebenen Menge Wasser zubereitet.

Werden die beiden Komponenten im Set als Flüssigkeiten bereit gestellt, kann deren Menge und Konzentration so gewählt sein, dass die Behandlungslösung ohne zusätzliches Abmessen durch einfaches Zusammengeben der beiden Komponenten direkt gebrauchsfertig hergestellt werden kann. Vorzugsweise kann aber, vor allem zur Volumen- und Gewichtsersparnis beim Transport, auch eine weitere Verdünnung flüssiger Komponenten in einer vorgelegten zusätzlichen Wassermenge vor Ort vorgesehen werden.

Die aus der ersten und zweiten Komponente erhaltene Behandlungslösung (Hygienisierungslösung) kann dann manuell mit einem an sich bekannten mobilen Sprühgerät oder auch automatisch mittels einer in vielen mobilen Trinkwasserbehältern integrierten Cleaning-in-Place-Anlage auf die trinkwasserberührten Oberflächen aufgetragen werden. Die Rohrleitungen, Pumpen und weitere Aggregate der mobilen Trinkwasseranlagen werden durch Füllen und/oder Umpumpen mit der Hygienisierungslösung behandelt. So ist sichergestellt, dass alle trinkwasserberührten Oberflächen mit der Hygienisierungslösung in Kontakt treten und behandelt werden. Weil die Komponenten des Sets rezepturgemäß kein Chlor, keine Chloride oder andere Chlorverbindungen enthalten, wird beim Hygienisierungsprozess kein Chlor frei. Das beauftragte Personal wird somit keinem giftigen Chlorgas ausgesetzt. Die Hygienisierungslösung wirkt aufgrund ihrer Chlor-/Chloridfreiheit nicht korrosiv für Edelstähle.

Nach der erforderlichen Einwirkzeit wird die verbrauchte Hygienisierungslösung mit Trinkwasser ausgespült, und das gesamte Spülwasser, das die unverbrauchten Komponenten der Hygienisierungslösung bzw. deren Reaktionsprodukte enthält, dadurch neutralisiert und deaktiviert, dass man es mit der dritten Komponente des Sets vermischt, z.B. durch Zugabe der dritten Komponente zu dem Spülwasser oder durch dessen Einleiten in eine vorgelegte Neutralisierungslösung.

Der pH-Wert des in der Regel noch sauren Spülwassers wird durch die alkalischen Anteile in der dritten Komponente in den für die Entsorgung günstigen neutralen pH-Werte-Bereich angehoben. Gleichzeitig reduziert das Reduktionsmittel in der dritten Komponente den Restgehalt an sauerstoffhaltigem Oxidationsmittel, z.B. den Wasserstoffperoxid-Gehalt des Spülwassers zu Wasser. Das neutralisierte Spülwasser kann danach schadlos entsorgt werden.

Nachfolgend wird die Erfindung durch ein Anwendungsbeispiel noch näher erläutert.

### Beispiel:

Zur Hygienisierung einer mobilen Trinkwasserspeicheranlage mit einem Volumen von 2 m³ wurde ein Set bestehend aus 2 kg einer ersten flüssigen Komponente, 2 kg einer zweiten flüssigen Komponente und 0,54 kg einer dritten, festen Komponente eingesetzt.

### Hierbei enthielt

A) die erste Komponente:
   3,5 Gew.-% Natriumhydrogensulfat,
   1,8 Gew.-% Schwefelsäure
   0,4 Gew.-% Phosphorsäure,
   Rest Wasser
B) die zweite Komponente:
   4,5 Gew.-% Wasserstoffperoxid
   Rest Wasser
C) die dritte Komponente:
   36 Gew.-% Natriumhydrogencarbonat
   64 Gew.-% Natriumsulfit.

Zur Herstellung der anwendungsbereiten Hygienisierungslösung wurden in 100 Liter Trinkwasser die erste und zweite Komponente gemeinsam gelöst. Die Lösung hatte einen pH-Wert von 2,1 und enthielt 860 ppm Wasserstoffperoxid. Mit einer Cleaning-in-Place-Anlage wurde diese Hygienisierungslösung auf die innenliegenden Flächen des Trinkwasserbehälters flächendeckend aufgetragen. Außerdem wurden die zum Trinkwasserbehälter gehörenden Rohrleitungen, Pumpen und Anlagenteile im Umlaufverfahren benetzt und gespült.

Nach einer Einwirkzeit von einer Stunde wurde die Hygienisierungslösung abgelassen und die abgelösten Ablagerungen und Verunreinigungen mit Trinkwasser ausgespült. Die Spülwässer wurden aufgefangen und mit der abgelassenen Hygienisierungslösung vereinigt, und die dritte Komponente wurde in den gesammelten Spülwässern gelöst.

Das so behandelte Spülwasser hatte einen pH-Wert von 6,3, und der Wasserstoffperoxid-Gehalt betrug < 1 ppm und konnte problemlos entsorgt werden. Die mobile Trinkwasserspeicheranlage und das darin nachgefüllte Trinkwasser waren nach der Behandlung hygienisch einwandfrei.

## Patentansprüche

1. Verfahren zur Hygienisierung von mobilen Trinkwasseraufbereitungs- und -speicheranlagen für die Bereitstellung von Trinkwasser einer hohen hygienischen Qualität, **dadurch gekennzeichnet, dass** man
a. ein Reagenzienset bereitstellt, das aus drei separaten Komponenten in stöchiometrisch aufeinander abgestimmten Mengen besteht, wobei
die erste Komponente eine saure feste Komponente oder saure flüssige Komponente in Form eines wässrigen Reinigungskonzentrats mit einem pH-Wert < 3 ist, die Mineralsäuren in Form der Mineralsäuren Phosphorsäure, Schwefelsäure und/oder Salpetersäure und/oder deren saure Salze enthält,
die zweite Komponente ein starkes halogenfreies Oxidationsmittel in Form von Alkalimetallpercarbonaten, -peroxiden und -perboraten oder von Wasserstoffperoxid in einer wässrigen Lösung enthält,
wobei man die Mengen der ersten und zweiten Komponente so wählt, dass bei ihrer Vermischung und Verdünnung, bezogen auf eine Menge von 100 l einer wässrigen Hygienisierungslösung, eine Hygienisierungslösung mit einem pH von weniger als 3, und mit einem Gehalt an aktivem Sauerstoff, der einem Wasserstoffperoxidgehalt von 650 bis 900 ppm in 100 l wässriger Hygienisierungslösung entspricht, erhalten wird
und die dritte Komponente in Kombination miteinander in wässriger Lösung alkalisch reagierende Substanzen sowie Reduktionsmittel zur vollständigen Reduktion des starken halogenfreien Oxidationsmittels der zweiten Komponente in der verbrauchten Hygienisierungslösung in fester oder flüssiger Form enthält, wobei in der dritten Komponente als in wässriger Lösung alkalisch reagierende Substanzen Alkalimetallhydroxide, -carbonate und/oder basische Alkalimetallphosphate, sowie als Reduktionsmittel Alkali- und Erdalkalisalze der dithionigen Säure, der schwefligen Säure, der dischwefligen Säure und der Thioschwefelsäure, enthalten sind,
b. die erste und die zweite Komponente unter Bildung einer wässrigen Hygienisierungslösung miteinander sowie gegebenenfalls mit einer zusätzlichen vorgegebenen Wassermenge vermischt,
c. die erhaltene wässrige Hygienisierungslösung auf die trinkwasserberührten Oberflächen und technischen Einrichtungen und Leitungen einer mobilen Trinkwasseranlage einwirken lässt,
d. die Hygienisierungslösung nach einer vorgegebenen Einwirkungszeit aus der mobilen Trinkwasseranlage entfernt und die Anlage unter im wesentlichen vollständiger Entfernung der Bestandteile der Hygienisierungslösung mit Wasser spült, und
e. die verbrauchte Hygienisierungslösung und gesammeltes Spülwasser aus Stufe d. vereinigt und durch Zugeben der dritten Komponente neutralisiert und anschließend entsorgt.

2. Verfahren nach Anspruch 1, **dadurch gekennzeichnet, dass** die mobile Trinkwasseraufbereitungs- und -speicheranlage in Krisengebieten oder in Gebieten ohne funktionierende lokale Infrastruktur oder zur Versorgung von Menschenansammlungen eingesetzt wird.

## Claims

1. Method for sanitizing mobile potable water preparation and storage systems for providing potable water of a high sanitary quality, **characterized in that**
a. a reagent set is provided which consists of three separate components in stoichiometrically matched amounts, wherein
the first component is an acidic solid component or acidic liquid component in the form of an aqueous cleaning concentrate with a pH <3, which contains mineral acids in the form of the mineral acids: phosphoric acid, sulfuric acid and/or nitric acid, and/or the acidic salts thereof,
the second component contains a strong halogen-free oxidizing agent in the form of alkali metal percarbonates, alkali metal peroxides and alkali metal perborates or of hydrogen peroxide in an aqueous solution,
wherein the amounts of the first and second components are selected in such a way that when mixed and diluted with respect to an amount of 100 l of an aqueous sanitizing solution, a sanitizing solution having a pH of less than 3 and an active oxygen content corresponding to a hydrogen peroxide content of 650 to 900 ppm in 100 l of aqueous sanitizing solution,
is obtained,
and the third component contains in combination substances which react alkaline in aqueous solution and reducing agents for the complete reduction of the strong halogen-free oxidizing agent of the second component in the used sanitizing solution in solid or liquid form, wherein the third component contains alkali metal hydroxides, alkali metal carbonates and/or basic alkali metal phosphates as substances which react alkaline in aqueous solution, and alkali metal salts and alkaline earth metal salts of the dithionous acid, of the sulfurous acid, of the disulfurous acid and of the thiosulfuric acid as reducing agents,
b. the first and second components are mixed together to form an aqueous sanitizing solution and are optionally mixed with an additional predetermined amount of water,
c. the resulting aqueous sanitizing solution is allowed to act on the surfaces in contact with potable water and on the technical equipment and pipes of a mobile potable water system,
d. the sanitizing solution is removed from the mobile potable water system after a predetermined exposure time and the system is rinsed with water thereby substantially completely removing the components of the sanitizing solution; and
e. the exhausted sanitizing solution and collected rinsing water from step d. are combined and neutralized by adding the third component and then disposed of.

2. Method according to claim 1, **characterized in that** the mobile potable water preparation and storage system is used in crisis areas or in areas without a functioning local infrastructure or for supplying crowds of people.

## Revendications

1. Procédé d'hygiénisation d'installations mobiles de traitement et de stockage d'eau potable pour fournir de l'eau potable d'une haute qualité hygiénique, **caractérisé en ce que**
a. on fournit un kit de réactifs qui est constitué de trois composantes séparées en quantités adaptées les unes aux autres sur le plan stoechiométrique, dans lequel
la première composante est une composante acide solide ou une composante acide liquide sous la forme d'un concentré de purification aqueux ayant un pH < 3 qui contient des acides minéraux sous la forme des acides minéraux que sont l'acide phosphorique, l'acide sulfurique et/ou l'acide nitrique et/ou leurs sels acides,
la seconde composante est un agent fortement oxydant sans halogène sous la forme de percarbonates, de peroxydes et de perborates de métal alcalin ou de peroxyde d'hydrogène dans une solution aqueuse,
on choisit les quantités de la première et de la seconde composante de telle sorte que lors de leur mélange et de leur dilution, on obtient, en relation par rapport à une quantité de 100 litres d'une solution d'hygiénisation aqueuse, une solution d'hygiénisation ayant un pH inférieur à 3 et une teneur en oxygène actif qui correspond à une teneur en peroxyde d'hydrogène de 650 à 900 ppm dans 100 litres de solution d'hygiénisation aqueuse,
et la troisième composante en combinaison mutuelle dans la solution aqueuse contient des substances à réaction alcaline ainsi que des agents réducteurs pour la réduction complète de l'agent fortement oxydant sans halogène de la seconde composante dans la solution d'hygiénisation consommée, sous forme solide ou liquide, la troisième composante contenant à titre de substances à réaction alcaline dans la solution des hydroxydes et des carbonates de métal alcalin et/ou des phosphates basiques de métal alcalin, ainsi qu'à titre d'agents réducteurs des sels alcalins et alcalino-terreux de l'acide dithionique, de l'acide sulfureux, de l'acide di-sulfureux et de l'acide thiosulfurique,
b. on mélange la première et la seconde composante l'une avec l'autre et le cas échéant avec une quantité d'eau donnée supplémentaire en formant une solution d'hygiénisation aqueuse,
c. on laisse réagir la solution d'hygiénisation aqueuse obtenue sur les surfaces et les installations techniques et les conduites d'une installation mobile d'eau potable, qui viennent en contact avec l'eau potable,
d. on enlève la solution d'hygiénisation hors de l'installation mobile d'eau potable après un temps d'action prédéterminé et on rince à l'eau l'installation en enlevant sensiblement complètement les composantes de la solution d'hygiénisation, et
e. on réunit la solution d'hygiénisation consommée et l'eau de rinçage collectée de l'étape d. et on la neutralise par addition de la troisième composante pour ensuite l'évacuer.

2. Procédé selon la revendication 1, **caractérisé en ce que** l'on utilise l'installation mobile de traitement et de stockage d'eau potable dans des régions de crise ou dans des régions sans infrastructure locale opérationnelle ou pour l'alimentation de rassemblements humains.
